Europäisches Patentamt

European Patent Office

Office européen des brevets

(11) Publication number: **0 447 808 A2**

## EUROPEAN PATENT APPLICATION

(21) Application number: 91102294.5

(22) Date of filing: 27.01.84

(51) Int. Cl.5: **C12N 15/30, A61K 39/015, C12N 15/62, C12P 21/00**

This application was filed on 18 - 02 - 1991 as a divisional application to the application mentioned under INID code 60.

(30) Priority: 28.01.83 AU 7843/83
10.06.83 AU 9788/83

(43) Date of publication of application:
**25.09.91 Bulletin 91/39**

(60) Publication number of the earlier application in accordance with Art.76 EPC: **0 134 799**

(84) Designated Contracting States:
**BE CH DE FR GB LI NL SE**

(71) Applicant: SARAMANE PTY. LTD.
**9 Moralla Road**
**Kooyong, VIC 3144(AU)**

(72) Inventor: **Kemp, David James**
**309 Belmore Road**
**North Balwyn, Victoria 3103(AU)**
Inventor: **Brown, Graham Vallancey**

**35 Walsh Street**
**Balwyn, Victoria 3103(AU)**
Inventor: **Anders, Robin Fredric**
**55 Brougham Street**
**North Melbourne, Victoria 3051(AU)**
Inventor: **Saint, Robert Bryce**
**2 Saville Court**
**Lower Templestone, Victoria 3107(AU)**
Inventor: **Coppel, Ross Leon**
**6 Mercer Road**
**Armadale, Victoria 3143(AU)**
Inventor: **Cowman, Alan Frederick**
**3/20 Earl Street**
**North Carlton, Victoria 3054(AU)**
Inventor: **Mitchell, Graham Frank**
**21 Sinclair Avenue**
**Lower Templestone, Victoria 3107(AU)**

(74) Representative: **Holmes, Michael John et al**
**Frank B. Dehn & Co. Imperial House 15-19**
**Kingsway**
**London WC2B 6UZ(GB)**

(54) Expression of Plasmodium falciparum polypeptides from cloned cDNA.

(57) DNA molecules comprising artificially constructed polynucleotide sequences substantially corresponding to all or a portion of Plasmodium falciparum messenger RNA (mRNA) or genomic DNA (gDNA). Especially DNA molecules comprising artificially constructed polynucleotide sequences coding for the whole or part of one or more proteins occurring in P. falciparum, or a precursor or modification thereof. Such DNA molecules are capable of being expressed as a polypeptide(s).

Synthetic peptides or polypeptides displaying the antigenicity of all or a portion of at least one P.falciparum antigen.

Compositions for stimulating immune responses against P.falciparum antigens in a mammal, comprising at least one polypeptide displaying the antigenicity of a P.falciparum antigen, together with a pharmaceutically acceptable carrier therefor.

EP 0 447 808 A2

EP 0 447 808 A2

## "EXPRESSION OF PLASMODIUM FALCIPARUM POLYPEPTIDES FROM CLONED CDNA"

This invention relates to DNA molecules comprising artificially constructed polynucleotide sequences substantially corresponding to all or a portion of Plasmodium falciparum messenger RNA (mRNA) or genomic DNA (gDNA), in particular it relates to polynucleotide sequences coding for at least one protein or part thereof. The invention especially relates to DNA molecules comprising artificially constructed polynucleotide sequences coding for the whole or part of one or more proteins occurring in P. falciparum, or a precursor or modification thereof. Such DNA molecules are capable of being expressed as a polypeptide(s).

Of the four species of Plasmodium causing malaria in man, P. falciparum is the most important being a major cause of infant mortality in tropical countries. It has been estimated that in Africa alone malaria causes one million deaths each year and that world-wide 2,000 million people live in areas where there is the potential for transmission of malaria parasites. Immunity to malaria develops slowly so that recurrent infections cause significant morbidity in children living in endemic areas. Adults who have grown up in endemic areas will often have detectable parasites in their blood but seldom suffer severe disease although malaria infection is a contributing cause to adult anaemia and may cause other serious illnesses.

In many countries malaria has become a more severe disease problem over the last decade. This is due to several factors which include the breakdown in malaria control programs, the emergence of insecticide resistance in the mosquito vectors and most importantly the emergence of strains of P. falciparum resistant to chloroquin, a key drug used in prevention and treatment of the infection.

P. falciparum is a single celled organism of the family, Plasmodiidae; order, Plasmodiida; subclass, Haemosporidia; subphylum, Sporozoa; and phylum, Protozoa. (Pathology of Protozoal and Helminthic Diseases. ed. R.A. Marcial-Rojas, Williams & Wilkins, Baltimore 1971)

Like other Plasmodia, P. falciparum has a complex life cycle. The infective stage to man (sporozoite) is injected into the blood when a female mosquito has a blood meal. Within minutes, sporozoites invade liver cells and within these cells undergo a stage of maturation and asexual multiplication resulting in the production of hundreds of merozoites from one sporozoite. Eventually the liver cell ruptures releasing merozoites which can then initiate a second asexual cycle in the blood by invading red blood cells (RBC).

As in the liver cell the parasite matures within the RBC and undergoes asexual multiplication to produce numerous merozoites which when released into the blood, upon rupture of the infected RBC, can invade another RBC and initiate another asexual cycle. For reasons that are not clearly understood, occasionally maturation within the RBC results in the formation of male or female gametes. When these sexual stages are ingested with a blood meal by a mosquito they fuse to initiate a process of sexual reproduction the end result of which is thousands of sporozoites in the salivary gland of the mosquito each capable of reinfecting a human being.

The immune response to malaria infection is complex. Both antibody and cell-mediated immune responses occur and responses are directed against components of several of the life cycle stages. The sera of putatively immune individuals contain antibodies recognizing over a hundred different polypeptide antigens in asexual blood stages of P. falciparum. Antibodies to asexual blood stage antigens do have anti- parasitic effects but which of the many specificities are host-protective is not firmly established.

There is a considerable body of evidence that indicates that the asexual blood stages of P.falciparum possess two types of antigens that may be targets of protective immune responses. Antigens of one type are common to all isolates of P.falciparum, thus immune responses with these specificities induced by infection with one isolate or strain of P.falciparum will be equally effective against other isolates or strains. However, antigens of the other type vary in different isolates or strains of P.falciparum. Thus, host-protective immune responses directed against such antigens will be restricted in their efficacy. One or more of these responses of restricted specificity accounts for the observation that exposure to an isolate of P.falciparum provides better protection against challenge with the homologous isolate than with heterologous isolates.

A system for the continuous culture of the asexual blood forms of P. falciparum in vitro is available, however, it will not be feasible to produce sufficient amounts of the appropriate antigens required for a vaccine from cultured parasites. Furthermore, parasites are grown in vitro in human RBCs and in a medium supplemented with human serum thus it would be difficult to ensure parasite antigens derived from cultures were free of human antigens which may induce autoimmunity in immunised individuals.

Using the techniques developed over the last ten years it is now possible to introduce the DNA (deoxyribonucleic acid) coding for non-bacterial proteins into bacterial cells via the intermediary of a plasmid or bacteriophage, (see for example Burrell, C.J. et al, Nature, 279, 43-47, 1979.). In general the construction of the recombinant DNA molecules comprises the steps of deriving the DNA template coding for the desired protein from the non-bacterial parent and inserting this piece of heterologous DNA into a

2

cloning vehicle, such as a bacteriophage, and then infecting an appropriate bacterial host with the modified bacteriophage. A general discussion of the manipulation of genes leading to the formation of recombinant DNA was published by S. Cohen in Scientific American, 233, 24-33, 1975.

Many non-bacterial genes have been inserted and multiplied within bacteria such as Escherichia coli, and many non-bacterial proteins have been expressed by bacteria using recombinant DNA technology, including the haemagglutinin of influenza viruses (Porter, A.G. et al, Nature, 282, 471-477, 1979) the hepatitis B virus protein (Burrell, C.J. et al, 1979, loc. cit.), and mouse immunoglobulin heavy chains (Kemp, D.J. & Cowman, A.F., Proc. Nat. Acad. Sci. U.S.A., 1981, 78, 4520-4524). Notwithstanding the considerable amount of work carried out in recent years on recombinant DNA research, there has been a paucity of results amenable to immediate and practical application in the field of recombinant DNA involving the manipulation of protozoan genetic material.

One approach to the production of vaccines for inducing immunity against Plasmodia antigens, disclosed in European Patent Applications Nos. 0062924 and 0071705, both in the name of The Wellcome Foundation Limited, has been based on the use of monoclonal antibodies to define certain antigens by molecular weight and immunofluorescence staining pattern, and to isolate these antigens for use in immunisation. The present invention, however, is based on an entirely different approach in using recombinant DNA techniques in the production of antigenic polypeptides.

The present invention provides for the synthesis of individual polypeptides substantially equivalent to naturally occurring Plasmodium proteins, in particular P. falciparum proteins recognised as antigens by defined sera. In one particular embodiment, this invention provides for the synthesis of polypeptides substantially equivalent to a family of Plasmodium antigens referred to as S antigens. S antigens are found in the sera of patients during the acute phase of a malaria infection and are also released into the medium when P.falciparum is cultured in vitro. Different isolates of P.falciparum are characterised by S antigens that may vary in antigenicity and molecular weight. Immune responses directed against S antigens may be one component of the host-protective isolate-restricted immunity referred to above. In a second embodiment, this invention provides for the synthesis of other polypeptides which are common to all isolates of P.falciparum and may therefore stimulate immune responses which are host-protective against all isolates.

The invention also provides for the synthesis of P. falciparum polypeptides either as individual entities or as fusion products of two or more polypeptide portions. In addition the present invention provides the means whereby the concurrent synthesis of immunogenic proteins normally encoded for by different variants of P. falciparum may be effected.

According to one aspect of the present invention, there is provided a DNA molecule comprising a nucleotide sequence substantially corresponding to all or a portion of P. falciparum RNA. Preferably, the nucleotide sequence codes for at least one polypeptide of P. falciparum. Such a DNA molecule is capable of being expressed as a polypeptide recognizable as substantially corresponding to a P. falciparum protein. In one particular aspect of the invention, the DNA molecule is capable of being expressed as a polypeptide recognisable as substantially corresponding to S antigen by its antigenic relationship to the S antigen released into culture supernatants by P.falciparum strains.

In an alternative embodiment, the nucleotide sequence may code for all or a portion of at least two P. falciparum polypeptides each derived from a different isolate, strain or variant of P. falciparum.

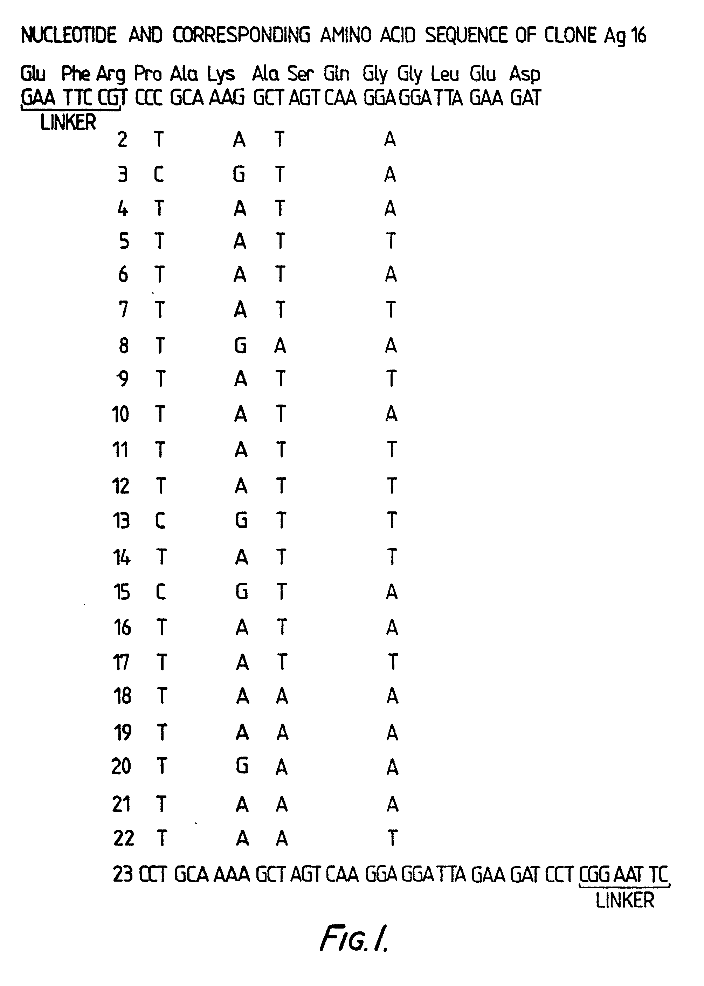

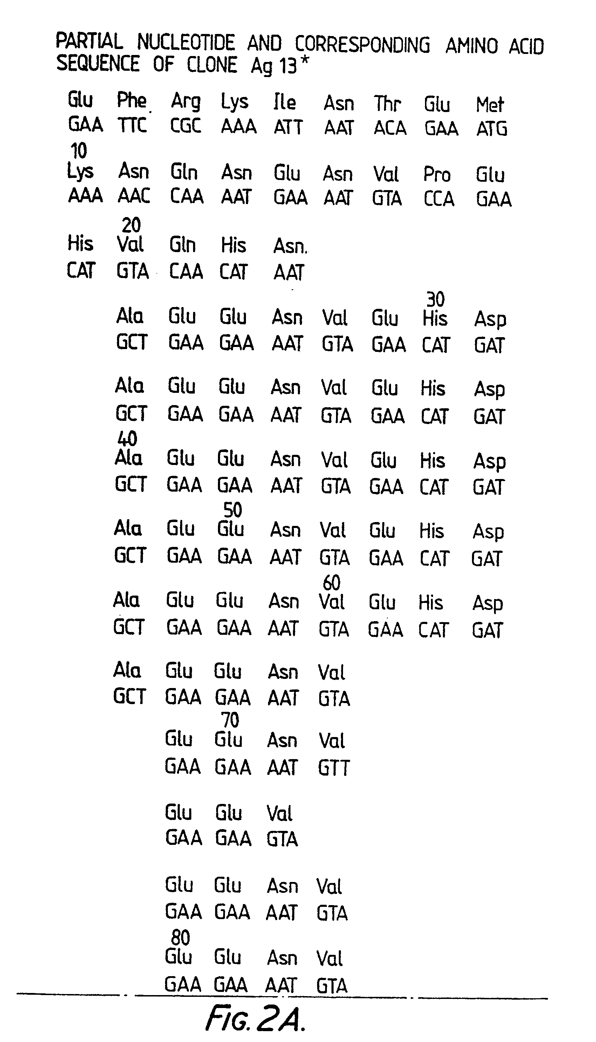

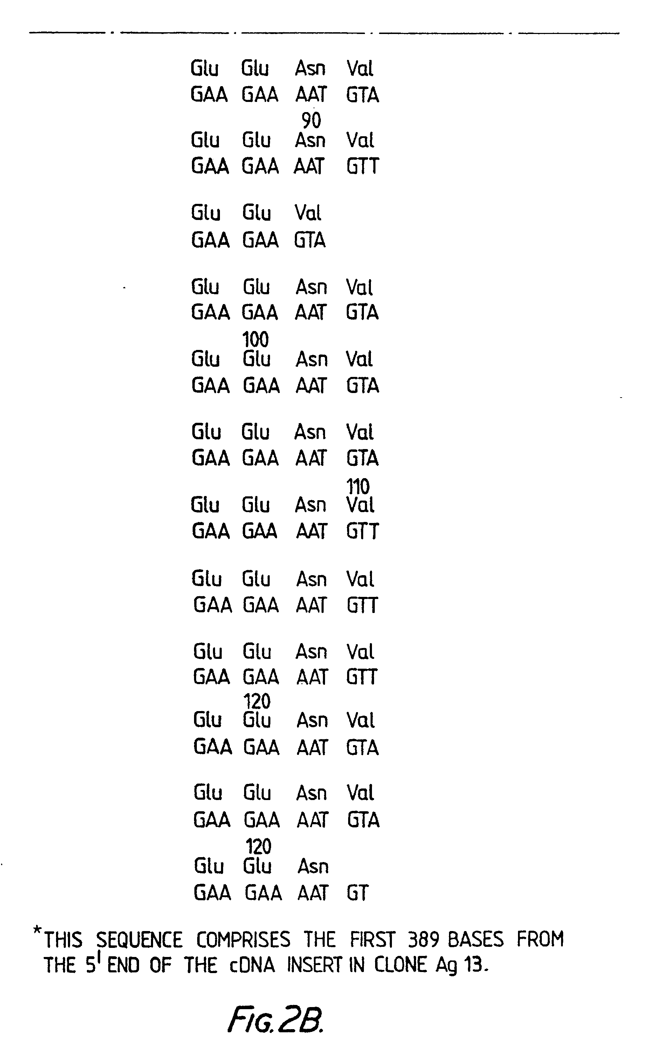

By way of exemplification of this aspect of the invention, the polynucleotide sequences may be characterised by at least a portion thereof having one of the partial base sequences substantially as shown in Figures 1 and 2 hereinafter. The particular sequence shown in Figure 1 has been identified as corresponding to a portion of the S antigen of P.falciparum FC27. The particular sequence shown in Figure 2 is capable of being expressed as a polypeptide with a relative molecular mass (Mr) of 140,000 which is common to all isolates of P.falciparum and is apparently, but not exclusively, located on the membrane of newly invaded red blood cells.

It will be appreciated that the polynucleotide sequences of this aspect of the invention may be obtained from natural, synthetic or semi-synthetic sources; furthermore, these polynucleotide sequences may be naturally-occurring sequences, or they may be related by mutation, including single or multiple base substitutions, deletions, insertions and inversions, to such naturally-occurring sequences, provided always that the DNA molecule comprising such a sequence is capable of being expressed as a polypeptide displaying the antigenicity of one or more antigens of P.falciparum.

The nucleotide sequence may have expression control sequences positioned adjacent to it, such control sequences being derived either from P.falciparum nucleic acid or from a heterologous source.

This invention also provides a recombinant DNA molecule comprising an expression control sequence having promoter sequences and initiator sequences as herein defined, and a nucleotide sequence substantially coding for all or part of at least one protein of P. falciparum, the nucleotide sequence being located 3' to the promoter and initiator sequences.

In yet another aspect, the invention provides a recombinant DNA cloning vehicle capable of expressing all or part of at least one protein of P. falciparum, comprising an expression control sequence having promoter sequences and initiator sequences, and a nucleotide sequence substantially coding for all or part of at least one protein of P. falciparum, the nucleotide sequence being located 3' to the promoter and initiator sequences.

In a further aspect, there is provided a host cell containing a recombinant DNA cloning vehicle and/or a recombinant DNA molecule as described above.

In yet further aspects, there are provided fused polypeptides comprising polypeptide sequences displaying P. falciparum antigenicity as the C-terminal sequence, and an additional polypeptide, for example a polypeptide coded for by the DNA of a cloning vehicle, as the N-terminal sequence fused thereto. Such a fused polypeptide can be produced by a host cell transformed or infected with a recombinant DNA cloning vehicle as described above, and it can be subsequently isolated from the host cell to provide the fused polypeptide substantially free of other host cell proteins.

The present invention also extends to synthetic peptides or polypeptides displaying the antigenicity of all or a portion of at least one antigen of P.falciparum. As used herein, the term "synthetic" means that the peptides or polypeptides have been produced by chemical or biological means, such as by means of chemical synthesis or by recombinant DNA techniques leading to biological synthesis. Such polypeptides can, of course, be obtained by cleavage of a fused polypeptide as described above and separation of the desired polypeptide from the additional polypeptide coded for by the DNA of the cloning vehicle by methods well known in the art. Alternatively, once the amino acid sequence of the desired polypeptide has been established, for example, by determination of the nucleotide sequence coding for the desired polypeptide, the polypeptide may be produced synthetically, for example by the well-known Merrifield solid-phase synthesis procedure [A.Marglin and R.B.Merrifield, Annu. Rev. Biochem. 39, 841 (1970)].

It will be appreciated that polypeptide sequences displaying antigenicity characteristic of antigens of P.falciparum will have utility in serological diagnosis, and in the preparation of single or multivalent vaccines against P.falciparum by methods well known in the art of vaccine manufacture.

The invention further provides a method of preparing a DNA molecule substantially coding for at least one polypeptide of P. falciparum comprising:

(a) isolating P. falciparum single stranded RNA;

(b) preparing a first single strand of DNA complementary to the single strand of P. falciparum RNA;

(c) preparing a second single strand of DNA complementary to and hydrogen bonded to the first DNA strand so as to produce a double strand of DNA.

The double strand of DNA so produced can be inserted into a cloning vehicle following digestion of the cloning vehicle with a restriction endonuclease, free ends of the cloning vehicle being joined to the DNA molecule so as to form a recombinant cloning vehicle. This in turn can be inserted into a suitable host cell by for example transformation or infection.

It will be appreciated also that the P. falciparum DNA inserted into the cloning vehicle and encoding polynucleotide sequences capable of being expressed as a polypeptide displaying antigenicity of one or more antigens of P. falciparum may be derived from chromosomal DNA of P. falciparum by digesting such DNA with an appropriate restriction endonuclease.

As used herein the terms listed below have the following meanings:-

Nucleotide: a unit of DNA or RNA comprising a sugar moiety (pentose), a phosphate and a nitrogenous heterocyclic base. The base is joined to the sugar moiety via the glycosidic carbon (1' carbon of the pentose) and the base characterizes the nucleotide. The four DNA bases are adenine (A), guanine (G), cytosine (C) and thymine (T). The four RNA bases are A, G, C and uracil (U).

Recombinant DNA: - a hybrid double stranded DNA sequence comprising at least two double stranded DNA nucleotide sequences, the first sequence not being found together in nature with the second sequence.

Cloning Vehicle:- non-chromosomal double stranded DNA capable of replicating when placed within a unicellular micro-organism.

Bacteriophage:- a cloning vehicle derived from viruses or bacteria which may infect certain strains of bacteria.

Plasmid:- a cloning vehicle derived from viruses or bacteria.

Structural Gene:- a sequence of DNA nucleotides which codes for a sequence of amino acids characteristic of a specific polypeptide.

Promoter Sequences:- sequences of DNA nucleotides which control the initiation, rate or magnitude of transcription.

Initiator Sequences:- sequences of DNA nucleotides which control the initiation of transcription.

EP 0 447 808 A2

Transcription:- the process whereby RNA polymerase is caused to move along the DNA sequence forming messenger RNA.

Translation:- the process of producing a polypeptide from messenger RNA.

Operon:- a structural gene(s) coding for polypeptide expression which is preceded by initiator sequences.

Expression:- the process involved in producing a polypeptide from a structural gene

Lysogeny:- the integration of bacteriophage nucleotide sequences into a bacterial genome.

The invention will be further described by way of reference to the accompanying diagrams, in which:

Figure 1 shows the nucleotide and corresponding amino acid sequence of a clone numbered as Ag16 which corresponds to the S antigen of isolate FC27.

Figure 2 shows a partial sequence of a clone numbered as Ag13 - the sequence has been arranged to show that this clone contains both 24 base repeats (8 amino acids) and 12 base repeats (4 amino acids).

Figure 3 is a schematic diagram of the construction of a bacteriophage vector and library from P. falciparum and expression of the polypeptides derived therefrom;

Figure 4 shows the detection with human immune sera of recombinant E. coli clones which are expressing polypeptides displaying P. falciparum antigenicity;

Figure 5 shows (A) a secondary screen using human immune sera of the positive clones selected in Figure 3, compared with (B) hybridization of $^{32}$P labelled cDNA from P. falciparum mRNA to a duplicate set of the same clones; and

Figure 6 shows the detection of the fused P. falciparum - β-galactosidase polypeptides by Coomassi staining (A), with anti-β-galactosidase antibodies (B) and also with human antibodies to malaria antigens (C).

Figure 7 shows an analysis by one-dimensional polyacrylamide gel electrophoresis of the protein composition of the clone (identified as clone Ag16 and containing the recombinant phage λAmp3-Ag16) expressing a polypeptide antigenically related to the S antigen of isolate FC27 (lane 2). The analysis of the semi-purified protein obtained by fractionating the insoluble pellet (lane 3) obtained from a bacterial lysate on Sepharose CL6B in the presence of 0.1% sodium dodecyl sulphate and 1 mM dithiothreitol is illustrated in lane 4. For comparison, lane 1 shows the analysis of the protein composition of a bacterial clone containing non-recombinant phage.

Figure 8 shows the detection of clone Ag16 with anitserum from a rabbit immunized with fusion protein of the clone Ag16. From a total of 78 antigen-positive clones on the same filter, clone Ag16 specifically reacts with the antiserum. This indicates that immunizing a rabbit with the fusion protein from clone Ag16 has induced antibodies to the P.falciparum component of the fusion protein.

Figure 9 shows in Panel A the detection of S antigens in the culture supernatants of P.falciparum isolate FC27 (lane 1) and cloned parasite line E12 (lane 2) with Papua New Guinea (PNG) serum containing antibodies to S antigens. The identical antigens are detected with antiserum from a rabbit immunized with fusion protein of the clone Ag16 (panel B).

Figure 10 (A to E) shows the specific staining of mature P.falciparum parasites when the rabbit antiserum prepared against the fusion protein from clone Ag16 is used in indirect immunofluorescence.

Figures 11 - 14 show the specific staining of P.falciparum blood-stage parasites when indirect immunofluorescence is performed using antisera prepared against clones Ag13, Ag23, Ag63 and Ag144 respectively.

General Approach

A variety of techniques are available for preparing the recombinant DNA molecule according to the invention, one of which comprises the steps of synthesising a single stranded DNA copy (cDNA) of the RNA purified from an isolate of whole P. falciparum using a reverse transcriptase enzyme. After the original RNA strand has been degraded the cDNA is converted into a double strand (ds cDNA), which is then treated to remove any loops of DNA which have formed using, for example, a nuclease enzyme. An alternative method of preparing the double stranded cDNA is via chemical synthesis using techniques well known in the art.

Once the double stranded cDNA has been produced the next step is to insert it into a cloning vehicle, which may be for example a bacterial plasmid or bacteriophage. This may be achieved by first cleaving the DNA of the purified cloning vehicle, for example λAmp 3 (described hereinafter), using a restriction endonuclease enzyme such as EcoRI, which cleaves the DNA at sites where complementary nucleotides are arranged in rotational symmetry. The double stranded P. falciparum cDNA can then be inserted between and linked to the open ends of the cloning vehicle by joining synthetic oligonucleotides to blunt ended ds cDNA and making the new termini cohesive by either exonuclease or endonuclease digestion,

5

prior to ligation with appropriately linearized cloning vehicle. Alternatively, other techniques well known in the art may be used.

Once the double stranded P. falciparum cDNA has been annealed with the DNA of the cloning vehicle, an appropriate host, such as a bacterium, is transformed, infected or lysogenized with the recombinant cloning vehicle, so as to permit that host to express the P. falciparum ds cDNA, and thereby produce a polypeptide or polypeptides which may display P. falciparum antigenicity.

There are several host-cloning vehicle combinations that could be used for the expression of P. falciparum polypeptides. For example useful cloning vehicles include bacterial plasmids such as pAT 153, (Twigg, A.J. Nature, 283, 216-218, 1980) pBR 322, (Sutcliffe, J. G. Cold Spring Harbour Symposium for Quantitative Biology, 43, 77-90, 1978) other E. coli plasmids and wider host range plasmids. Bacteriophages such as the many derivatives of phage λ, and particularly λAmp 3,may also be suitable. Hosts that may be used include bacteria such as strains of E. coli K. 12, e.g. E. coli HB101, (Boyer, H. W. et al, J. Mol. Biol., 41, 459-472, 1969) E. coli χ1776, (Curtiss, R. et al, Ann Report Dep. of Microbiology University of Alabama,1976, 96-106) E. coli χ2282 and E. coli MRC1, strains of Bacillus subtilus and Pseudomonas, as well as yeasts and other fungi, and other unicellular organisms. Alternatively, eucaryotic cells such as mammalian cells may be used. It is only to be expected, however, that not all hosts will be equally effective.

Within each cloning vehicle, various sites may be available for insertion of the P. falciparum ds cDNA, each site being designated by the restriction endonuclease enzyme which cleaves DNA. Thus, for example, enzyme EcoRI cleaves bacteriophage λAmp 3 in the gene coding for β-galactosidase.

The selection of the site on the cloning vehicle for insertion of P. falciparum ds cDNA may be governed by a variety of factors, for example size of polypeptide to be expressed and location of promoter and initator sequences. Consequently not all sites may be equally effective for a given polypeptide.

It is essential that the P. falciparum ds cDNA inserted into the cloning vehicle can be read in the correct phase. In order to achieve this it may be necessary to insert supplementary nucleotides for example between the start points of transcription and translation of the P. falciparum ds cDNA fragment whose expression is desired. Addition of such nucleotides must not, of course, form a nucleotide sequence that could interrupt translation.

Expression of P. falciparum ds cDNA, which has been inserted into a cloning vehicle, which in turn has been used to transform, infect or lysogenize a suitable host cell, may be detected by the appearance of a function specific for the protein, that is, immunological activity in the case of P. falciparum. Several methods are available, for example the essentially immunological colony screening method disclosed by D. J. Kemp and A. F. Cowman in Proc Nat. Acad. Sci. USA, 1981, 78, 4520-4525. One alternative technique is to inject into a laboratory animal the crude bacterial extract or purified fused polypeptide derived from a culture of bacteria transformed with an appropriately engineered cloning vehicle and to test for the formation of appropriate antibodies. A second alternative is to perform an immunoprecipitation of a crude extract of the bacterial cells. Yet a further method is the "Maxicell Technique" described by Sancar et al in J. Bact., 1979, 137, 692-693.

The nature of the polypeptide produced as a result of expression by the host of the recombinant DNA molecule of the invention will depend on the point of insertion into the DNA of the cloning vehicle, so that in practice a fused polypeptide may be formed which comprises a polypeptide coded for by P. falciparum ds cDNA and an additional polypeptide coded for by the DNA of the cloning vehicle. Thus, for example, if the bacteriophage λAmp 3 is cleaved by EcoRI a fused polypeptide comprising a portion of the β-galactosidase enzyme and the polypeptide coded for by the P. falciparum ds cDNA may be expressed. The fused polypeptide may then be selectively cleaved so as to separate the desired P. falciparum polypeptide from the superfluous amino acid sequence. Cleavage may be effected outside the host following harvest of the microbial culture by techniques well known to those skilled in the art. Cleavage may be necessary in order that the P. falciparum expression product can exert the desired antigenic activity, however during harvest of the microbial culture the fact that a superfluous amino acid sequence is linked to the required P. falciparum polypeptide may help to prevent degradation of the expression product by endogenous enzymes. Alternatively cleavage may be effected within the host, this may be achieved by inserting into the cloning vehicle, DNA coding for the desired cleavage enzymes.

The production of a fusion product of a polypeptide coded for by P. falciparum ds cDNA and a portion of for example β-galactosidase enzyme may increase the stability of the hybrid protein in E. coli and even enhance the immunogenicity of the P. falciparum protein.

Alternatively, appropriate nucleotide sequences, derived for example from P. falciparum RNA, may be inserted before the P. falciparum ds cDNA so as to ensure that the P. falciparum ds cDNA can be expressed alone, and not as a fusion product with a host polypeptide.

Once host cells or recombinant DNA cloning vehicles containing at least some P. falciparum ds cDNA have been identified, as explained above, the recombinant cloning vehicle DNA may be purified and then analysed in order to determine how much of the P. falciparum ds cDNA has been inserted. In order to do this the recombinant cloning vehicle DNA is treated with several different restriction endonucleases, for example Eco RI, Pst 1, Sal 1, Bgl II, Bam H1, Hind III and Hinf 1, and the digestion products may be analysed by gel electrophoresis, followed by sequencing of the fragments using the method described by Maxam and Gilbert in Proc. Nat. Acad. Sci. USA, 1977, 74, 560-564.

The various DNA molecules may be useful as a probe for the in vitro diagnosis of the presence in biological samples of P. falciparum, and in particular may be used to define the isolate causing an outbreak of malaria. For this purpose the DNA molecules may be labelled, in known manner, with a radioactive isotope.

Once recombinant DNA cloning vehicles expressing polypeptides corresponding to natural P.falciparum immunogens have been identified, the expressed polypeptides synthesized by these cloning vehicles, for example, as a fusion protein with β-galactosidase, can be isolated substantially free of contaminating host cell components by techniques well known to those skilled in the prior art.

Isolated proteins containing, in part, amino acid sequences corresponding to a portion or all of a natural immunogen of P.falciparum may be used to raise monospecific but polyclonal antisera by immunizing rabbits, mice or other animals using well established procedures.

In addition the expression product of the recombinant cloning vehicle may be used for serological diagnosis and in the preparation of single or multivalent vaccines against P. falciparum by methods well known in the art of vaccine manufacture. Such vaccines are effective in stimulating antibodies in vaccinated animals and thereby protecting against plasmodia.

Traditional vaccine formulations may comprise the antigenic polypeptides together with known adjuvants such as aluminium hydroxide, in association with a pharmaceutically acceptable carrier. Suitable carriers include liquid media such as saline solution appropriate for use as vehicles to introduce the polypeptides into a patient.

An alternative vaccine formulation may comprise a virus or microorganism in association with a pharmaceutically acceptable carrier, the virus or microorganism having inserted therein a DNA molecule according to this invention for stimulation of an immune response directed against polypeptides encoded by the inserted DNA molecule.

Further characteristics and features of the invention are decribed in the following Examples which are presented by way of illustration only and are not to be considered as limiting the scope of the present invention in any way.

EXAMPLE 1 The Production of cDNA Clones which Express Natural Immunogens of P.falicparum.

Reagents.

DNA polymerase I, EcoR1, and Xbal from Boehringer Mannheim GmbH. EcoR1 methylase, BstN1, exonucleases Bal 31 and S1, calf intestinal phosphatase and T4 ligase were from New England Biolabs, Bethesda, Md. Reverse transcriptase was the gift of J. Beard, Life Sciences Inc., St. Petersburg, Fla. EcoR1 linkers (CGGAATTCCG) were from New England Biolabs, Xbal linkers (CTCTAGAG) were from Amersham, England. β-galactosidase (chromatographically purified) was from P. L. Biochemicals Inc., Milawukee, Wis.

Strains.

Bacteriophage strains λgt11 and E.coli strains RY1073, and RY1082 were the generous gifts of T. Huynh, R. Young and R. Davis (Stanford). P. falciparum mRNA.

P. falciparum isolate FCQ27/PNG (FC27) obtained from the Madang area of Papua New Guinea was cultured in vitro (Chan, P. et al SE Asian J Trop. Med. Public Health 11:435-440 1980). Enriched parasite preparations were prepared by lysis of the erythrocytes with Saponin (Zuckerman, A. et al Bull W.H.O. 37:431-436 1967). RNA was prepared from them and poly A$^+$ RNA selected by oligo dT cellulose chromatography.

Enzyme reactions.

The procedures were as in Maniatis et al "Molecular Cloning", Cold Spring Harbour, 1982.

## Construction of cDNA clones in λgt10.

mRNA (~ 1μg) was copied into cDNA with reverse transcriptase and double-stranded cDNA was then prepared with DNA polymerase I. After treatment with nuclease S1 the ds cDNA was methylated with EcoR1 methylase and ligated to 0.5 μg of phosphorylated Ecor1 linkers with T4 ligase. The mixture was then treated with 20 units of EcoR1 for 90 min. at 37° and fractionated by electrophoresis on a 1% agarose gel in 50mM Tris, 20 mM NaOAc 2mM EDTA pH 8.2. DNA between 0.6 and 2.0 kb was recovered by electrophoresis on to Schleicher and Schuel NA45 membrane filter and elution in 1M NaC1, 50mM free base arginine at 70°C for 1 hr followed by ethanol precipitation and aliquots (20 ng) were ligated to EcoR1-cleaved λgt10 DNA (1 μg), packaged into phage and plated on E. coli RY1073 (Young R, and Davis R, Proc. Natl. Acad. Sci. USA, in press). About 2 x $10^5$ recombinants were obtained, and ~40% of these hybridized detectably to $^{32}$P-cDNA from P. falciparum.

## Construction of Amp3.

DNA (5 μg) from pBR322 was cleaved with EcoR1 and BstN1. After treatment with 0.3 units of exonuclease Bal31 for 30 sec the DNA was ligated to Xba linkers as above, cleaved with 100 units of Xbal and fractionated on a 1% agarose gel. The ~1.7kb fragment was recovered and ligated to 1 μg of Xbal cleaved gt11 (Young, R. and Davis, R. Proc. Natl. Acad. Sci. USA, in press) DNA and packaged as above. E. coli RY1082 was infected with the phage and plated on L plates containing ampicillin (30 μg/ml). An Amp R colony was chosen and shown to be lysogenic for a lac$^+$, temperature sensitive phage (designated λAmp3) identical to λgt11 except that it contained a ~1.7 kb fragment inserted into the Xbal site.

## Insertion of cDNA from λgt10 into λAmp3.

DNA (500 μg) prepared from plate stocks of the λgt10-cDNA library grown on E. coli (600 $r_k^-$,$m_k^+$) was cleaved with EcoR1 (1000 units) for 2 hrs at 37° and 1/50 of the total was labelled with $^{32}$P-dATP and the Klenow fragment of DNA polymerase I. After phenol extraction, the labelled and unlabelled DNAs were mixed and centrifuged for 18 hr at 37,000 RPM on a 10-40% glycerol gradient in 10mM Tris/HC1 pH 7.4, 1mM EDTA and 300 mM NaOAc. The cDNA fragments were detected as a radioactive peak well separated from the vector fragments and recovered by ethanol precipitation.

Uncut λAmp3 DNA (50 μg in 100 μl) was ligated for 4 hr at 15° to seal and protect the cohesive ends from subsequent phosphatase treatment. The ligase was then inactivated at 70° for 10 min, NaCl and Tris/HCl pH 7.4 were added to 50mM and 100mM and the DNA cleaved with EcoR1 (500 units) for 4hr. The DNA was treated with calf intestinal phosphatase (0.02 units), phenol extracted and ethanol precipitated. Aliquots (1μg) were ligated for 16 hr at 15° to a 4 fold molar excess of the inserts from the λgt10 library in a 10 μl system and packaged as above. E. coli RY1082 was then infected with phage in the packaging mix for 30 min at 30°, incubated at 30° for a further 30 min after adding L broth (1 ml) to allow expression of β lactamase and plated on nitrocellulose filters on L plates containing 30 μg/ml ampicillin at 30°. Approximately 5 x $10^4$ Amp R colonies were obtained from 6 μg vector.

## Screening of the λAmp3 - P. falciparum cDNA library with antibodies to P. falciparum.

Colonies were replicated to nitrocellulose filters [Hanahan, D. and Meselson, M. Gene 10, 63-67 (1980)] grown for 1-2 hr at 30° on CY plates containing 30 μg/ml ampicillin and induced at 42° for 1.5 hr. The colonies were lysed by placing the filters on 3MM paper saturated with 1% NaDodSO$_4$ in H$_2$0 for 15 min and then moved into an atmosphere saturated with CHCl$_3$ for a further 15 min. The filters were washed by rocking in 10mM Tris-HC1, 0.15M NaCl, pH8.4, 3% bovine serum albumin (BSA) for 2 hr and then Tris:NaCl alone for 1 hr at room temperature. They were then treated with affinity purified antibodies to P. falciparum - (25 μg/ml) in BSA:Tris:NaCl for 2 hr, washed twice in Tris:NaCl and twice in Tris:NaCl containing 0.05% Triton X-100, treated with $^{125}$I-labelled protein A from S.aureus (40 μCi/μg; 0.4 μCi/ml) in BSA:Tris:NaCl for 1 hr, washed, dried and auto-radiographed with an intensifying screen for 16 hr.

## Preparation and purification of sera.

Serum was collected with informed consent from adults free of P. falciparum in the endemic Madang area of Papua New Guinea. IgG isolated on Protein A-Sepharose from each serum was tested for inhibition of growth of P. falciparum in vitro (Brown, G.V. et al, Infect. Immun. 39: in press 1983) and five inhibitory sera were pooled. The antibodies were purified by two cycles of absorption of P. falciparum (isolate FC27) proteins bound to CNBr-activated Sepharose. Saponin enriched parasites were sonicated in PBS and centrifuged at 2,000g for 10 min. The supernatant was adjusted to approximately 20mg/ml with PBS and conjugated to CNBr-Sepharose. Bound antibodies were eluted with 0.1M glycine, 0.15M NaCl, pH 2.6 and immediately adjusted to pH 7.4 with 2M Tris-HCl pH7.4.

Analysis of proteins by Western Blotting

Protein extracts of induced λAmp3 - P. falciparum clones were prepared and fractionated on 7.5% acrylamide NaDodSO$_4$ gels. Proteins from the gels transferred electrophoretically to nitrocellulose and incubated at room temperature in BSA:Tris:NaCl 1 hr before reaction for 90 min with rabbit anti-β-galactosidase or anti-malaria antibodies. Antibodies to E. coli in both reagents were removed by incubating 100 μl of rabbit serum or 0.5 to 1.0 mg of human IgG with 1 ml of sonicate of λAmp3 infected E. coli for 1 hr at 4°C, followed by centrifugation (12,000g, 10 min) and the supernatant diluted with BSA:Tris:NaCl to 50 ml. The nitrocellulose sheets were then washed, reacted with $^{125}$I-labelled protein A, rewashed and autoradiographed as above. Rabbit antiserum to β-galactosidase was prepared with commercially available enzyme (P-L Biochemicals Inc., lot 535-3). Injection of 0.5 mg β-galactosidase in complete Freund's adjuvant (subcutaneously and intramuscularly) was followed at 4 week intervals by 0.5 mg in incomplete Freund's adjuvant. Serum collected 2 weeks after the last injection was used after absorbing out antibodies to antigens in E. coli RY1082 as above.

## RESULTS AND DISCUSSIONS

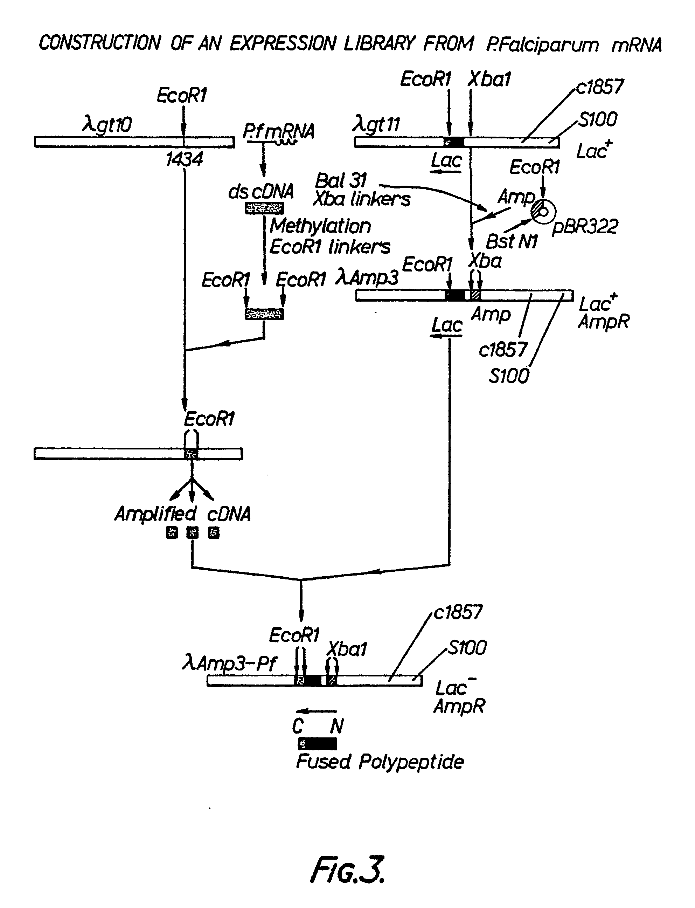

Our strategy for construction of an expression library from P. falciparum mRNA, based on the λgt11 expression system of Young and Davis is shown in Fig. 3. cDNA cloned into the unique EcoR1 site near the 3' end of the β-galactosidase gene of λgt11 can be expressed as polypeptides fused to β-galactosidase. E. coli colonies lysogenic for the temperature sensitive recombinants can then be grown at the permissive temperature followed by induction of the phage at 42° to achieve high levels of expression. High-frequency-lysogeny E. coli strains were employed to ensure that most colonies obtained after infection with phage are lysogens. However, a significant proportion of the colonies are always non-lysogens which being lac⁻, are difficult to distinguish from recombinant lysogens. We introduced the β-lactamase gene from pBR322 into λgt11 (see Fig. 3 and Materials and Methods), generating a phage designated λAmp3. Lysogens of λAmp3-cDNA give ampR-lac⁻ colonies readily distinguishable from parental amp R-lac + colonies on plates containing ampicillin and 5-bromo-4-chloro-3-indolyl-β-D-galactoside, while non-lysogens are killed. Further, λAmp3 lysogens can be directly selected from cells infected with phage from a packaging reaction, eliminating the need to amplify a phage stock.

To prevent religated parental molecules predominating in our library the EcoR1-cut λAmp3 vector was treated with phosphatase. Because this markedly reduced the cloning efficiency, we first amplified the cDNA by cloning it into the phage vector λgt10 (Fig.3). DNA from the λgt10-cDNA phage pool was cleaved with EcoR1 and the released cDNA was isolated and ligated to phosphatase-treated EcoR1 cleaved λAMP3 DNA to generate the final expression library. This procedure resulted in a library of ~5 x 10⁴ lysogenic Amp R colonies of which ≧98% were lac⁻ and >50% hybridized detectably to $^{32}$p cDNA from P. falciparum.

Identification of E. coli colonies which express P. falciparum antigens

To detect colonies expressing antigens of P. falciparum we lysed induced colonies in situ with NaDodSO$_4$ and chloroform vapour under conditions which allow binding of proteins directly to the nitrocellulose. After treatment with BSA the filters were treated with antibodies followed by $^{125}$I-labelled protein A from S. aureus which binds to antibodies localized at the site of a colony. Similar procedures have recently been decribed.

Antibodies from immune individuals in the endemic Madang area of Papua New Guinea were used to detect P. falciparum antigens. The IgG fractions of the individual sera which inhibited growth of P. falciparum in vitro were pooled. The IgG pool contained antibodies against many P. falciparum proteins, but also reacted strongly with E. coli, necessitating enrichment of the antibodies by affinity chromatography on P. falciparum proteins and depletion of the anti E. coli activity (Materials and Methods).

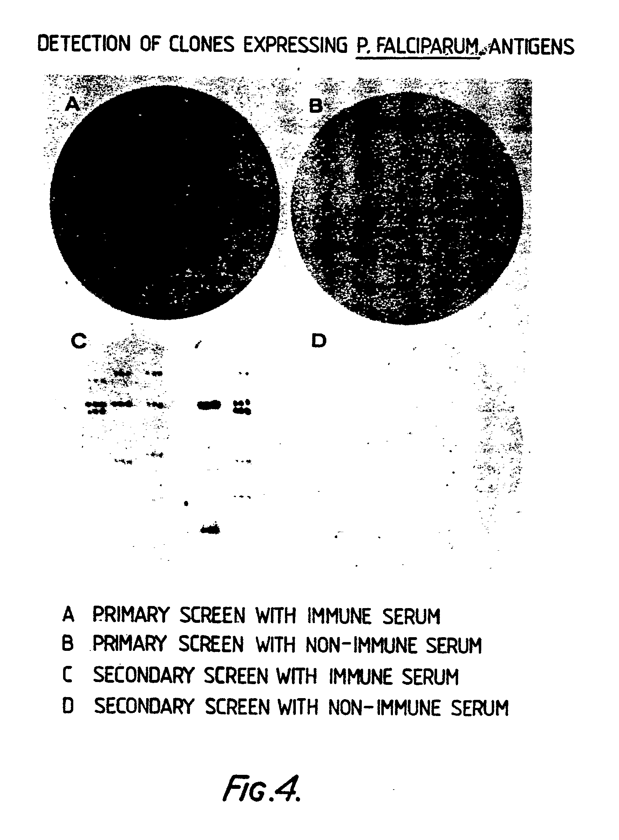

The result of such a screen on a filter containing about 10³ λAmp3-cDNA colonies is shown in Fig. 4. Several colonies have apparently reacted with the IgG, giving signals of varying intensity. No such variation is seen with normal IgG although both IgGs give a background reaction with all colonies. Clones in the area of the signals (generally 3-10 colonies) were plated for single colonies and picked at random in triplicate, so that some should be positive with the remainder providing negative controls. A significant proportion again reacted with the immune IgG but not with normal IgG. The signals were reproducible in the triplicates and the intensity of positive signals in any row (all of which derive from the same original positive) was characteristic.

The antigen-positive colonies contain P. falciparum cDNA sequences

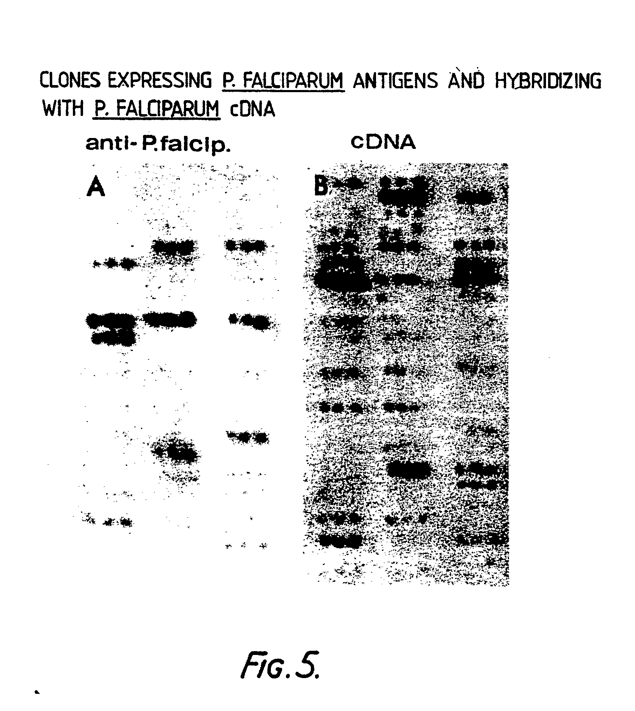

We tested a replica of the colonies shown in Fig 5A by colony hybridization with $^{32}$P cDNA to establish whether the positive clones contained P. falciparum cDNA sequences. All of the antigen-positive clones hybridized (Fig. 5B). The extent varied markedly and there was no relationship between the extents of immunological reactivity and hybridization. Most appear to be derived from mRNAs of moderate abundance. The differences in extent of hybridization indicates that the positive colonies derive from different mRNAs.

P. falciparum antigens expressed as polypeptides fused to $\beta$-galactosidase.

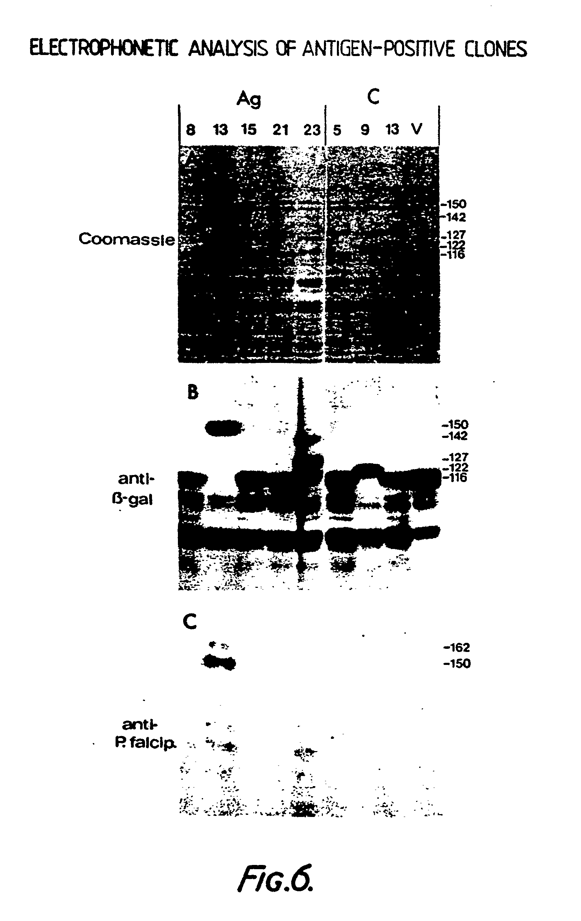

We examined proteins from lysates of induced antigen-positive and negative clones by gel electrophoresis. Staining with Coomassie blue revealed that some clones, both antigen-positive and negative, produced abundant quantities of unique polypeptides larger than $\beta$-galactosidase (e.g. clones Ag13, Ag23 and C9 in Fig. 6). When the proteins were transferred from gels to nitrocellulose by the "Western blotting" procedure and scored with anti-$\beta$-galactosidase serum, these unique large polypeptides all reacted strongly (Fig. 6). Polypeptides of lower molecular weight were also evident. These may be degradation products or they may be generated by internal initiation or premature termination.

When Western blots were scored with the affinity-purified anti-P. falciparum IgG, the results fell into 3 classes (Fig. 6). Firstly, the large polypeptide in clone Ag13 reacted strongly, demonstrating that it contains a P. falciparum antigen sequence as well as $\beta$-galactosidase and hence is an antigenic fused polypeptide. No reaction was observed with a similar large $\beta$-galactosidase containing polypeptide in the antigen-negative clone C9 (Fig. 6). Hence this clone expresses a fused polypeptide which is not recognized as an antigen. In other antigen-positive clones, for example clone Ag23 in Fig. 6, the large polypeptide reacted and in addition there was a smear of smaller, presumably degraded material. This polypeptide is apparently unstable in E. coli RY1082, even though this strain contains the 1on⁻ mutant which reduced degradation.

In a third class of clones, no reactive polypeptides were demonstrable with immune human IgG. This may be the result of extensive degradation, or may reflect inability of the polypeptide to retain antigenicity after boiling in NaDodSO₄. We cannot rule out the possibility that these latter clones are false-positives in the colony screening system.

EXAMPLE 2 The Production of P.falciparum S antigens by Recombinant DNA Technology.

Of 78 antigen-positive E.coli colonies produced as described above and initially examined, one (Ag16) contained a large, particularly abundant fusion protein.

Isolation of fusion protein from clone Ag16

E.coli lysogenic for the Amp3-Ag16 recombinant phage were grown in L-broth up to a density of $OD_{600}$ = 0.6 at a temperature of 30°. The phage was induced by incubating the cultures at 45° for 15 min. Following this induction the cultures were incubated for a further 90 min at 39° and the bacteria were then harvested by centrifugation for 10 min at 3,000 x g.

Bacteria harvested from 1 litre cultures were resuspended in 20 ml Tris-buffer (10 mM Tris-HCl, pH 8.0, 2 mM EDTA, 50 mM NaCl) and lysed by the addition of 0.25 mg/ml lysozyme and incubating for 30 min. To the bacterial lysate was added Triton X-100 (final concentration 0.2%), an equal volume of Tris buffer, containing 20 mM MgCl₂ and 1 μg/ml DNase (Deoxyribonuclease, Grade I, Boehringer Mannheim, FRG). After incubating at room temperature for 10 min cell debris was pelleted by centrifugation at 4,000 x g. The 4,000 x g supernatant was then centrifuged for 30 min at 40,000 x g to sediment the insoluble fusion protein together with other particulate material. The pellet was solubilised in 5 ml of 0.1 M NaPO₄ buffer, pH 6.4, containing 2% sodium dodecyl sulphate (SDS) and 10 mM dithiothreitol (DTT) by boiling for 2 min and loaded on to a column of Sepharose CL6B (Pharmacia, Uppsala, Sweden) which was equilibrated and eluted with 0.2 M NaPO₄ buffer, pH 6.4 containing 0.1% SDS and 1 mM DTT. Aliquots of the fractions eluting from the Sepharose column were examined by 1-dimensional SDS polyacrylamide gel electrophoresis (PAGE). Those containing the fusion protein were pooled, desalted by passage over a PD10 dialysing column (Pharmacia) equilibrated with 1 mM $\beta$-mercaptoethanol in $H_2O$ and freeze dried.

## Preparation of Antisera

Rabbits were injected subcutaneously and intramuscularly with freeze dried fusion protein (0.5 mg) suspended in 1 ml phosphate buffered saline (PBS) and emulsified in an equal volume of Freund's complete adjuvant. The rabbits received a second injection 4 weeks later with 0.5 mg of the same antigen emulsified in Freund's incomplete adjuvant and were bled for antiserum 2 weeks later.

Antisera were also raised in mice. In this case the bacteria from approximately 1 ml. of an induced culture were lysed in phosphate buffered saline by 4 cycles of freezing and thawing, emulsified in an equal volume of Freund's complete adjuvant and injected intraperitoneally and subcutaneously into female BALB/c mice. The mice received a second injection 4 weeks later with the same amount of bacterial lysate without added adjuvant, and subsequently bled for antiserum.

## Immunoblotting (Western Blotting)

Antigens released into the culture medium by P.falciparum isolates growing in vitro were fractionated on one-dimensional SDS-polyacrylamide gels (SDS-PAGE), electrophoretically transferred to nitrocellulose and probed with antibodies raised against the fusion protein or PNG serum essentially using the procedures described by Burnette W.N.,1981, Analyt. Biochem. 112, pp 195-203.

## Immunofluorescence

Antiserum raised against the fusion protein isolated from clone Ag16 (rabbit anti-Ag16 serum) was tested for reactivity against P.falciparum isolate FC27 by using the procedure of indirect immunofluorescence. Air dried, thin smears of parasitized red blood cells were fixed in acetone/methanol (90:40, v:v) for 15 min at room temperature. The dried smears were reacted with appropriately diluted (1:100 to 1:1000) rabbit anti-Ag16 serum for 30 min at room temperature. After three 15 min washes in PBS the smears were incubated with a 1:40 dilution of fluorescein-conjugated sheep anti rabbit immunoglobulin for 30 min at room temperature. After three 15 min washes in PBS the slides were flooded with 90% glycerol and then examined under a microscope using ultraviolet illumination.

## Nucleic Acid Sequencing

The cDNA insert of clone Ag16 was subcloned into the phage vector M13.mp9 (Messing J., Crea R., and Seeburg P.H. 1981, Nucl. Acids. Res., 9, pp 209-321), and sequenced according to the dideoxynucleotide chain termination method (Sanger F., Coulson A.R., Barrell B.G., Smith A.J.H., and Roe B.A., 1980, J. Mol. Biol., 143, pp 167-178).

## RESULTS AND DISCUSSION

## Characterisation of Clone Ag16

Using the procedures described in Example 1, clone Ag16 was identified as an antigen-positive E.coli colony which contained an abundant large fusion protein. The recombinant phage λAmp 3-Ag16 contains a P.falciparum cDNA insert of 830 base pairs. The base sequence of clone Ag16 is shown in Fig.1 and indicates that the antigen coded for by this partial polynucleotide sequence has a homologous repeat structure of 11 amino acids tandemly repeated 23 times.

## Isolation of Ag16 Fusion Protein

This fusion protein was insoluble and could be enriched for by lysing induced organisms and centrifuging down insoluble material. This pellet was solubilized by heating in a buffer containing 2% SDS and 10 mM DTT and a single passage over a Sepharose CL6B gel filtration column provided fusion protein free of the majority of proteins in the original E.coli lysate (Fig.7).

## Clone Ag16 corresponds to the S Antigen of P.falciparum FC27.

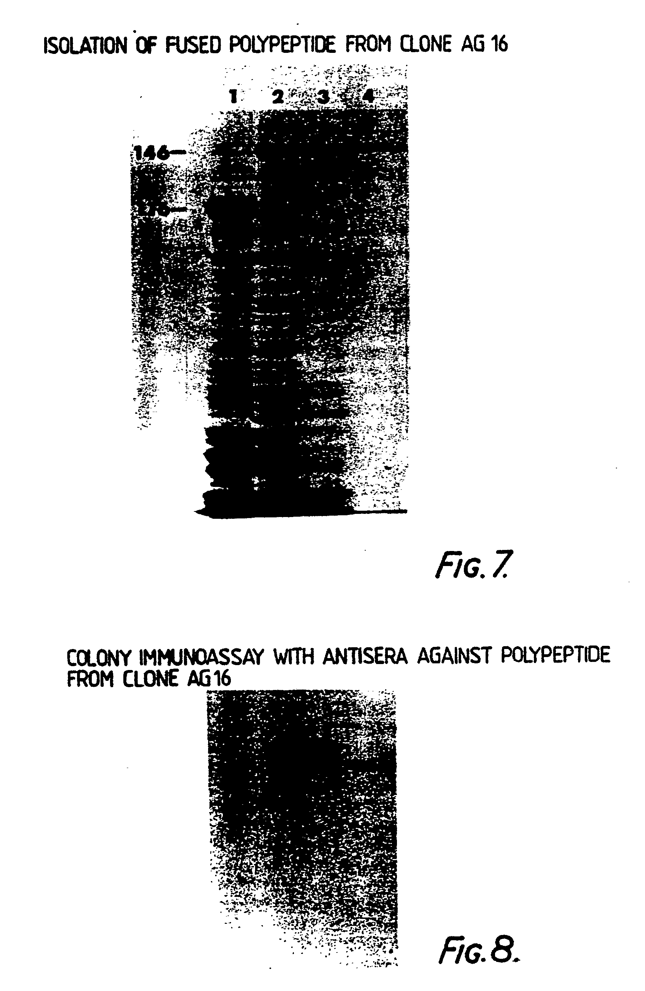

A rabbit immunized with the semi-purified fusion protein obtained from clone Ag16 produced antibodies that reacted with clone Ag16 and not other E.coli colonies containing either other recombinant bacteriophage or the non-recombinant phage λAmp 3 (Fig.8). Thus this fusion protein induced an antibody response primarily directed against determinants expressed in the P.falciparum portion of the protein.

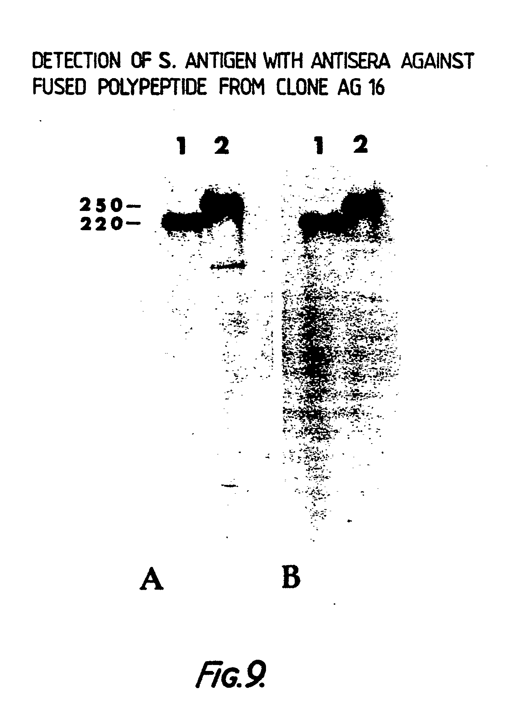

Conclusive evidence that the Ag16 clone corresponded to a portion of the isolate FC27 S antigen was obtained by Western blotting experiments. Isolate FC27 has an S antigen (Mr 220,000) which is the dominant antigen detected when culture supernatants are analysed by the Western blotting procedure using selected sera from naturally immune Papua New Guineans. One clonal population (E12) of P.falciparum derived from isolate FC27 by limit dilution culture expresses a larger but antigenically related S antigen (Mr approximately 250,000). When equivalent culture supernatants were analysed by the Western blotting procedure using anti-Ag16 serum as the probe the S antigens of isolate FC27 and clone E12 were the only antigens detected (Fig.9).

## Association of the Antigen with Mature Parasites

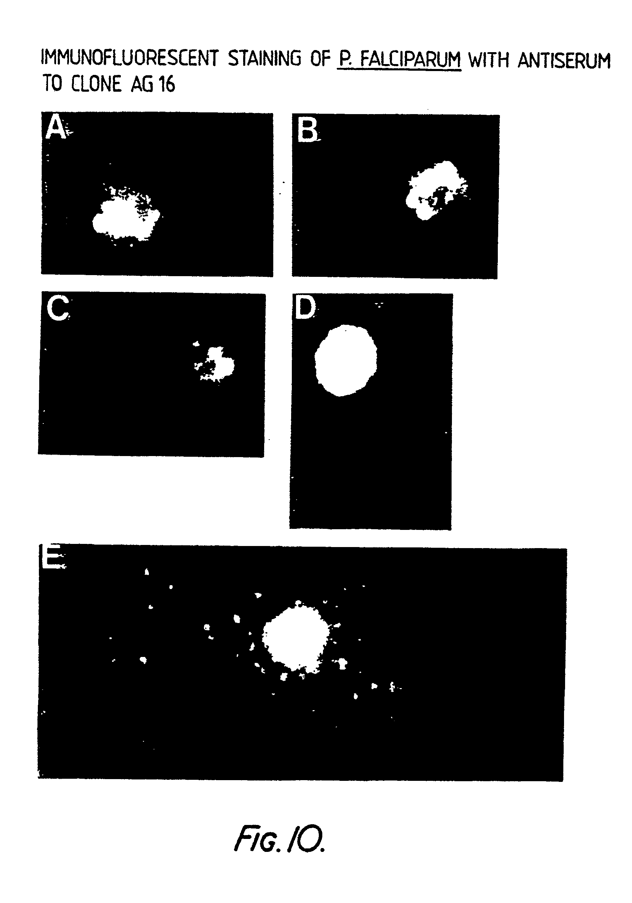

Anti-Ag16 serum was used in indirect immunofluorescence experiments to demonstrate that the corresponding antigen in isolate FC27 was associated with mature asexual stages in the red blood cell. The staining pattern (Fig.10) in which the merozoites inside the segmented schizont were clearly outlined is consistent with the S antigen being located within the parasitophorous vacuole and perhaps also on the merozoite surface.

## EXAMPLE 3 The Production of Other Natural Malaria Immunogens.

The processes used in Example 1 for preparing recombinant bacteria phage and for selecting E.coli clones making malaria antigens, and in Example 2 for characterising one clone with respect to the parasite antigen it corresponds, to were repeated so as to identify clones producing other antigens of P.falciparum.

## Results

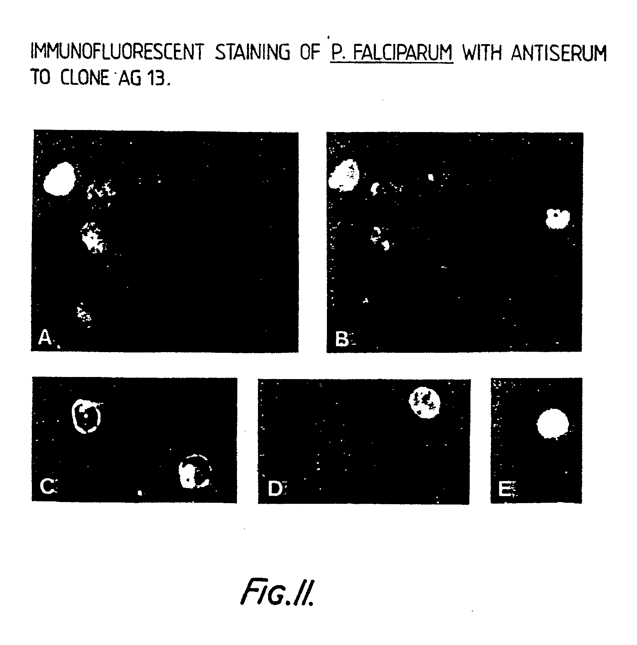

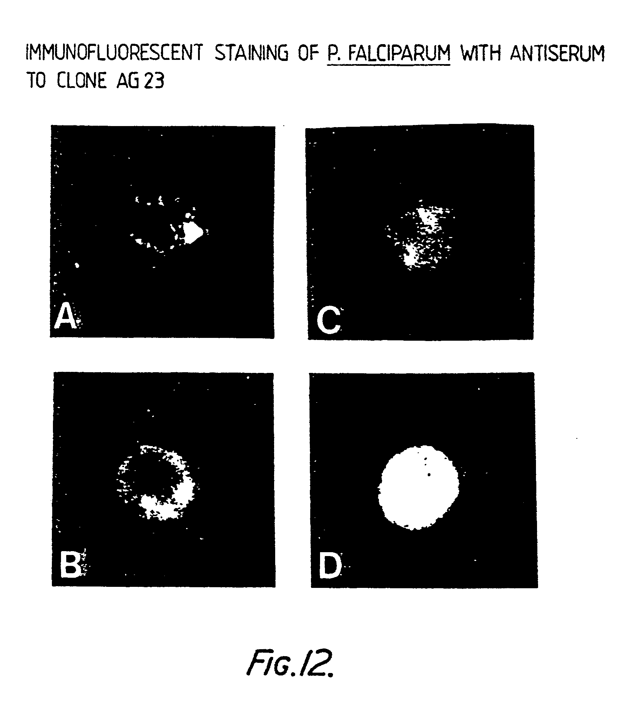

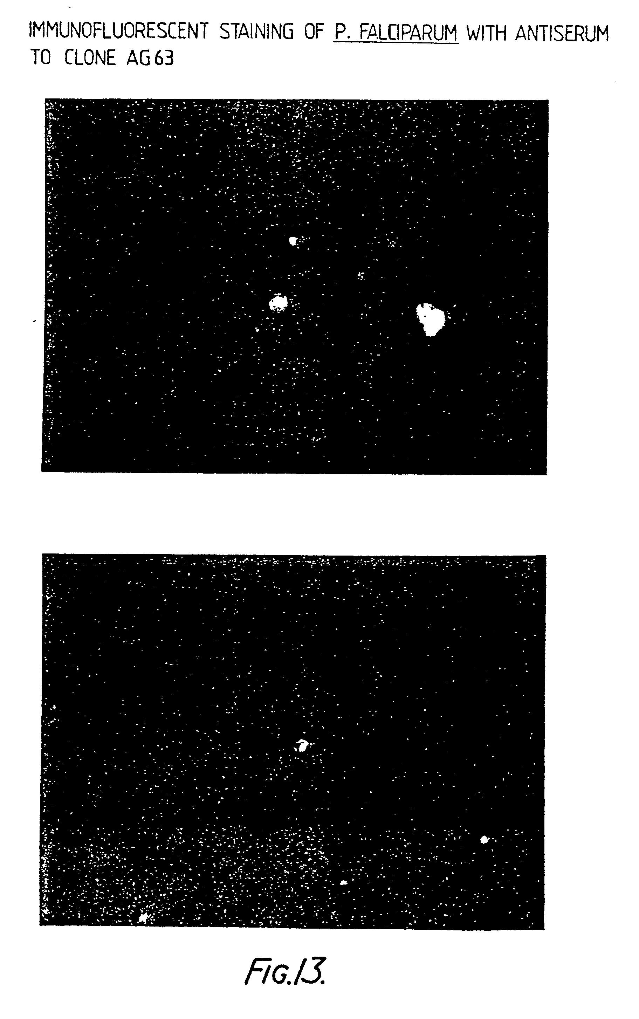

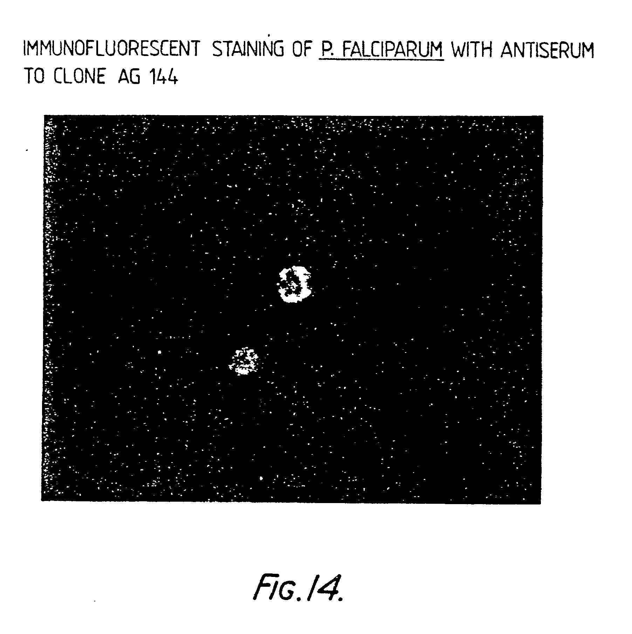

Available nucleotide sequence data on one of these additional clones is given in Figure 2. Information concerning the molecular weight, stage and strain specificity and ultrastructural localisation of these antigens is given in Figures 11-14 and in Tables 1 - 4.

## Table 1.

### Molecular Weight of P.falciparum (Isolate FC27) Proteins Corresponding to Antigen-Positive Clones

| Clone | Molecular Weight[*] |
|---|---|
| Ag 7 | 155,000 & 210,000 |
| Ag 13 | 155,000 & 210,000 |
| Ag 23 | 120,000 [§] |
| Ag 63 | 75,000 |
| Ag 144 | 112,000 |

[*] Molecular weights are approximate as they were determined by the electrophoretic mobility of the proteins on sodium dodecyl sulphate polyacrylamide gels relative to the mobility of proteins of known size and should be more appropriately referred to as relative molecular mass (Mr).

[§] An antigenically cross-reactive protein is present in isolates K-1 (Thailand) and NF7 (Ghana) but differs slightly in size. The K-1 antigen is Mr $\cong$127,000 and the NF7 antigen is Mr $\cong$118,000.

## Table 2.

Stage Specificity of P.falciparum (Isolate FC27) Proteins Corresponding to Antigen-Positive Clones

|        | Ring | Trophozoite | Schizont |
|--------|------|-------------|----------|
| Ag 7   | +    | −           | +        |
| Ag 13  | +    | −           | +        |
| Ag 23  | −    | +           | +        |
| Ag 63  | +    | −           | +        |
| Ag 144 | ND*  | ND          | ND       |

* ND = Not done

## Table 3.

Isolate Specificity of P.falciparum Proteins Corresponding to Antigen-Positive Clones

|         | FC27 | K-1 | NF7 |
|---------|------|-----|-----|
| Ag 7    | +    | +   | +   |
| Ag 13   | +    | +   | +   |
| Ag 23 * | +    | +   | +   |
| Ag 63   | +    | +   | +   |
| Ag 144  | +    | +   | +   |

* Ag 23 varies in apparent MW in the 3 isolates (see Table 1).

## Table 4

Patterns of Immunofluorescent Staining Seen with Antisera
Raised to Antigen-Positive Clones.*

Ag 7    The antigen is located on the surface of cells infected with ring-stage
        (immature)parasites.   Some staining was also evident in mature
        schizont-infected cells.

Ag 13   Essentially the same staining pattern as for Ag 7.  In some cases the
        antigen appeared to be evenly distributed over the surface of erythro-
        cytes infected with ring stages.  In other erythrocytes, also infected
        with ring stages, patches of more intense fluorescence were evident.
        Surface location of Ag 13 was demonstrated by reacting serum with intact
        cells.  Antiserum bound only to the surface of infected cells.

Ag 23   Late trophozoites and schizonts expressed this antigen.  Fluorescence
        of trophozoites was associated with a large number of "speckles"
        distributed over the entire parasitized cell.  There were often two
        small circular areas of more intense fluorescence, especially seen in
        the late trophozoite/early schizont.  At the late schizont stage,
        intense fluorescence was seen over the whole parasitized cell.

Ag 63   An antiserum to Ag 63 reacted predominantly with cells infected with
        mature parasites (cells containing malaria pigment).  Staining was
        evident around the pigment and over the entire parasite or as a
        network round developing merozoites.  Weaker staining was evident
        with very immature parasites where a ring of fluorescence surrounded
        the parasite within the red cell.

Ag 144  Only mature parasites were stained.  Fluorescence was visible either
        over the whole parasite, or as patches or as a network of fluorescence
        outlining the segmented schizont.

* The indirect immunofluorescent antibody procedure was used in each case.
Primary antisera were derived from rabbits or mice.  The secondary reagents
used were FITC-conjugated sheep anti-mouse immunoglobulin or FITC-conjugated
sheep anti-rabbit immunoglobulin.  Parasitized cells fixed to glass slides
were used as antigens.

## Claims

1. A recombinant DNA molecule comprising an artifically constructed nucleotide sequence substantially corresponding to all or a portion of a P. falciparum mRNA, wherein said nucleotide sequence codes for at least one polypeptide displaying the antigenicity of an antigenic polypeptide of an asexual blood stage of P. falciparum or an antigenic portion thereof, which antigenic polypeptide or portion thereof is immunogenic in humans.

2. A recombinant DNA molecule as claimed in claim 1, wherein said nucleotide sequence codes for at least one polypeptide displaying the antigenicity of an S antigen of P. falciparum or an antigenic portion thereof.

3. A recombinant DNA molecule as claimed in claim 1, wherein said nucleotide sequence codes for at least one polypeptide displaying the antigenicity of at least an antigenic portion of an antigen selected from antigens of P. falciparum FC27 having the following characteristics:
   a) Mr about 155,000 or about 210,000 and located predominantly at the surface of erythrocytes infected with ring-stage P. falciparum FC27; or
   b) Mr about 120,000 and expressed during the late trophozoite and schizont-stage of P. falciparum FC27; or
   c) Mr about 75,000 and expressed predominantly by mature asexually-produced P. falciparum FC27; or
   d) Mr about 112,000 and expressed by mature asexually produced P. falciparum FC27.

4. A recombinant DNA molecule as claimed in claim 1 or claim 2, wherein said nucleotide sequence is ligated at the 5' end to a second nucleotide sequence coding for an additional peptide or polypeptide.

5. A recombinant DNA molecule as claimed in any one of claims 1 to 4, wherein said nucleotide sequence coding for at least one polypeptide displaying the antigenicity of an antigenic polypeptide of an asexual blood stage of P. falciparum or an antigenic portion thereof is operatively linked to an expression control sequence.

6. A recombinant DNA molecule as claimed in any one of claims 1 to 5 which is a vector capable of transforming and replicating in a microorganism.

7. A vector as claimed in claim 6, wherein the nucleotide sequence coding for at least one polypeptide displaying the antigenicity of an antigenic polypeptide of an asexual blood stage of P. falciparum or an antigenic portion thereof is inserted in a bacteriophage.

8. An expression vector capable of transforming and replicating in a microorganism, wherein a nucleotide sequence coding for at least polypeptide displaying the antigenicity of an antigenic polypeptide of an asexual blood stage of P. falciparum or an antigenic portion thereof is operatively linked to an expression control sequence.

9. A microorganism containing a recombinant DNA molecule as claimed in any one of claims 1 to 5 or a vector as claimed in any one of claims 6 to 8.

10. A polypeptide having an N-terminal Met residue obtainable by growing a microorganism transformed by a vector as claimed in any one of claims 6 to 8 under conditions whereby said polypeptide is produced.

11. A fused polypeptide comprising an artificially constructed polypeptide sequence displaying the antigenicity of an antigenic polypeptide of an asexual blood stage of P. falciparum, or antigenic portion thereof, joined at the N-terminus to an additional peptide or polypeptide, wherein said antigenic peptide or portion thereof is immunogenic in humans.

12. A method for preparing a peptide or polypeptide displaying the antigenicity of an antigenic polypeptide of an asexual blood stage of P. falciparum or an antigenic portion thereof, which comprises the steps of culturing a microorganism containing a recombinant DNA molecule as claimed in any one of claims 1 to 8 under conditions whereby said peptide or polypeptide is synthesised and recovering said peptide or polypeptide thus produced.

13. An artificially constructed peptide or polypeptide which displays the antigenicity of all or a portion of an S antigen of P. falciparum which is immunogenic in humans.

14. An artificially constructed polypeptide which displays the antigenicity of all or a portion of an antigen selected from antigens of P. falciparum FC27 which are immunogenic in humans and which have the following characteristics:

   a) Mr about 155,000 or about 210,000 and located predominantly at the surface of erythrocytes infected with ring-stage P. falciparum FC27; or

   b) Mr about 120,000 and expressed during the late trophozoite and schizont-stage of P. falciparum FC27; or

   c) Mr about 75,000 and expressed predominantly by mature asexually-produced P. falciparum FC27; or

   d) Mr about 112,000 and expressed by mature asexually produced P. falciparum FC27.

15. A method of preparing a fused polypeptide as claimed in claim 11 which comprises the steps of culturing a microorganism as claimed in claim 9 capable of expressing said polypeptide under conditions whereby said polypeptide is synthesized and recovering said polypeptide thus produced.

16. A method of producing a polypeptide displaying the antigenicity of an antigenic polypeptide of an asexual blood stage of P. falciparum or an antigenic portion thereof which method comprises cleaving a fused polypeptide as claimed in claim 11 so as to release said additional peptide or polypeptide.

17. A composition for stimulating an immune response in a mammal against one or more P. falciparum antigens comprising at least one artifically constructed peptide or polypeptide as claimed in claim 13 or claim 14, or a peptide or polypeptide prepared by a method as claimed in claim 12, together with a pharmaceutically acceptable carrier therefor.

18. A composition for stimulating an immune response in humans against one or more P. falciparum antigens comprising a virus or microorganism in association with a pharmaceutically acceptable carrier, said virus or microorganism having inserted therein a DNA molecule comprising a nucleotide sequence capable of expressing at least one polypeptide displaying the antigenicity of an antigenic polypeptide of an asexual blood stage of P. falciparum or an antigenic portion thereof, which antigenic polypeptide or portion thereof is immunogenic in humans.

19. Use of a peptide or polypeptide as claimed in claim 13 or claim 14, or a peptide or polypeptide prepared by a method as claimed in claim 12, for the manufacture of a composition for stimulating an immune response in a mammal against one or more P. falciparum antigens.

20. A fused polypeptide comprising a polypeptide displaying the antigenicity of an antigenic polypeptide of an asexual blood stage of P. falciparum or an antigenic portion thereof fused to a further molecule, wherein said antigenic polypeptide or portion thereof is immunogenic in humans.

21. A fused polypeptide comprising a polypeptide displaying the antigenicity of an antigenic polypeptide of an asexual blood stage of P. falciparum or an antigenic portion thereof fused to a further peptide or polypeptide, a solid support or an amino acid coded for by an initiator codon, wherein said antigenic polypeptide or portion thereof is immunogenic in humans.

22. A method of producing a polypeptide displaying the antigenicity of an antigenic polypeptide of an asexual blood stage of P. falciparum or an antigenic portion thereof, which antigenic polypeptide or portion thereof is immunogenic in humans, characterised in that said polypeptide synthesised by total chemical synthesis on a solid-phase support.

NUCLEOTIDE AND CORRESPONDING AMINO ACID SEQUENCE OF CLONE Ag 16

| Glu | Phe | Arg | Pro | Ala | Lys | Ala | Ser | Gln | Gly | Gly | Leu | Glu | Asp |
|-----|-----|-----|-----|-----|-----|-----|-----|-----|-----|-----|-----|-----|-----|
| GAA | TTC | CGT | CCC | GCA | AAG | GCT | AGT | CAA | GGA | GGA | TTA | GAA | GAT |

<u>GAA TTC CGT</u> LINKER

| | | | | | | | | | | | | |
|---|---|---|---|---|---|---|---|---|---|---|---|---|
| 2 | T | | | A | | T | | | | | | A |
| 3 | C | | | G | | T | | | | | | A |
| 4 | T | | | A | | T | | | | | | A |
| 5 | T | | | A | | T | | | | | | T |
| 6 | T | | | A | | T | | | | | | A |
| 7 | T | | | A | | T | | | | | | T |
| 8 | T | | | G | | A | | | | | | A |
| 9 | T | | | A | | T | | | | | | T |
| 10 | T | | | A | | T | | | | | | A |
| 11 | T | | | A | | T | | | | | | T |
| 12 | T | | | A | | T | | | | | | T |
| 13 | C | | | G | | T | | | | | | T |
| 14 | T | | | A | | T | | | | | | T |
| 15 | C | | | G | | T | | | | | | A |
| 16 | T | | | A | | T | | | | | | A |
| 17 | T | | | A | | T | | | | | | T |
| 18 | T | | | A | | A | | | | | | A |
| 19 | T | | | A | | A | | | | | | A |
| 20 | T | | | G | | A | | | | | | A |
| 21 | T | | | A | | A | | | | | | A |
| 22 | T | | | A | | A | | | | | | T |

23 CCT GCA AAA GCT AGT CAA GGA GGA TTA GAA GAT CCT <u>CGG AAT TC</u>
LINKER

*FIG. 1.*

PARTIAL NUCLEOTIDE AND CORRESPONDING AMINO ACID
SEQUENCE OF CLONE Ag 13*

| Glu | Phe | Arg | Lys | Ile | Asn | Thr | Glu | Met |
|-----|-----|-----|-----|-----|-----|-----|-----|-----|
| GAA | TTC | CGC | AAA | ATT | AAT | ACA | GAA | ATG |

10
| Lys | Asn | Gln | Asn | Glu | Asn | Val | Pro | Glu |
|-----|-----|-----|-----|-----|-----|-----|-----|-----|
| AAA | AAC | CAA | AAT | GAA | AAT | GTA | CCA | GAA |

20
| His | Val | Gln | His | Asn. |
|-----|-----|-----|-----|-----|
| CAT | GTA | CAA | CAT | AAT |

30
| Ala | Glu | Glu | Asn | Val | Glu | His | Asp |
|-----|-----|-----|-----|-----|-----|-----|-----|
| GCT | GAA | GAA | AAT | GTA | GAA | CAT | GAT |

| Ala | Glu | Glu | Asn | Val | Glu | His | Asp |
|-----|-----|-----|-----|-----|-----|-----|-----|
| GCT | GAA | GAA | AAT | GTA | GAA | CAT | GAT |

40
| Ala | Glu | Glu | Asn | Val | Glu | His | Asp |
|-----|-----|-----|-----|-----|-----|-----|-----|
| GCT | GAA | GAA | AAT | GTA | GAA | CAT | GAT |

50
| Ala | Glu | Glu | Asn | Val | Glu | His | Asp |
|-----|-----|-----|-----|-----|-----|-----|-----|
| GCT | GAA | GAA | AAT | GTA | GAA | CAT | GAT |

60
| Ala | Glu | Glu | Asn | Val | Glu | His | Asp |
|-----|-----|-----|-----|-----|-----|-----|-----|
| GCT | GAA | GAA | AAT | GTA | GAA | CAT | GAT |

| Ala | Glu | Glu | Asn | Val |
|-----|-----|-----|-----|-----|
| GCT | GAA | GAA | AAT | GTA |

70
| Glu | Glu | Asn | Val |
|-----|-----|-----|-----|
| GAA | GAA | AAT | GTT |

| Glu | Glu | Val |
|-----|-----|-----|
| GAA | GAA | GTA |

| Glu | Glu | Asn | Val |
|-----|-----|-----|-----|
| GAA | GAA | AAT | GTA |

80
| Glu | Glu | Asn | Val |
|-----|-----|-----|-----|
| GAA | GAA | AAT | GTA |

FIG. 2A.

```
Glu   Glu   Asn   Val
GAA   GAA   AAT   GTA
                90
Glu   Glu   Asn   Val
GAA   GAA   AAT   GTT

Glu   Glu   Val
GAA   GAA   GTA

Glu   Glu   Asn   Val
GAA   GAA   AAT   GTA
      100
Glu   Glu   Asn   Val
GAA   GAA   AAT   GTA

Glu   Glu   Asn   Val
GAA   GAA   AAT   GTA
                110
Glu   Glu   Asn   Val
GAA   GAA   AAT   GTT

Glu   Glu   Asn   Val
GAA   GAA   AAT   GTT

Glu   Glu   Asn   Val
GAA   GAA   AAT   GTT
      120
Glu   Glu   Asn   Val
GAA   GAA   AAT   GTA

Glu   Glu   Asn   Val
GAA   GAA   AAT   GTA
      120
Glu   Glu   Asn
GAA   GAA   AAT   GT
```

*THIS SEQUENCE COMPRISES THE FIRST 389 BASES FROM THE 5' END OF THE cDNA INSERT IN CLONE Ag 13.

# FIG.2B.

CONSTRUCTION OF AN EXPRESSION LIBRARY FROM P.Falciparum mRNA

FIG.3.

DETECTION OF CLONES EXPRESSING P. FALCIPARUM ANTIGENS

A  PRIMARY SCREEN WITH IMMUNE SERUM
B  PRIMARY SCREEN WITH NON-IMMUNE SERUM
C  SECONDARY SCREEN WITH IMMUNE SERUM
D  SECONDARY SCREEN WITH NON-IMMUNE SERUM

Fig.4.

CLONES EXPRESSING <u>P. FALCIPARUM</u> ANTIGENS AND HYBRIDIZING
WITH <u>P. FALCIPARUM</u> cDNA

anti- P.falcip.          cDNA

*FIG.5.*

ELECTROPHONETIC ANALYSIS OF ANTIGEN-POSITIVE CLONES

FIG.6.

ISOLATION OF FUSED POLYPEPTIDE FROM CLONE AG 16

**Fig. 7.**

COLONY IMMUNOASSAY WITH ANTISERA AGAINST POLYPEPTIDE
FROM CLONE AG16

**Fig. 8.**

DETECTION OF S. ANTIGEN WITH ANTISERA AGAINST
FUSED POLYPEPTIDE FROM CLONE AG 16

**Fig.9.**

IMMUNOFLUORESCENT STAINING OF P. FALCIPARUM WITH ANTISERUM
TO CLONE AG 16

FIG. 10.

IMMUNOFLUORESCENT STAINING OF P. FALCIPARUM WITH ANTISERUM TO CLONE AG 13.

*Fig.II.*

IMMUNOFLUORESCENT STAINING OF P. FALCIPARUM WITH ANTISERUM
TO CLONE AG 23

FIG. 12.

IMMUNOFLUORESCENT STAINING OF <u>P. FALCIPARUM</u> WITH ANTISERUM
TO CLONE AG 63

*FIG. 13.*

IMMUNOFLUORESCENT STAINING OF P. FALCIPARUM WITH ANTISERUM
TO CLONE AG 144

Fig. 14.